(19) Europäisches Patentamt
European Patent Office
Office européen des brevets

(11) EP 4 499 027 B1

(12) **EUROPEAN PATENT SPECIFICATION**

(45) Date of publication and mention of the grant of the patent:
**25.02.2026 Bulletin 2026/09**

(21) Application number: **23716368.8**

(22) Date of filing: **03.03.2023**

(51) International Patent Classification (IPC):
***A61K 8/26*** *(2006.01)* ***A61K 8/31*** *(2006.01)*
***A61K 8/41*** *(2006.01)* ***A61K 8/58*** *(2006.01)*
***A61K 8/891*** *(2006.01)* ***A61Q 1/10*** *(2006.01)*

(52) Cooperative Patent Classification (CPC):
**A61Q 1/10; A61K 8/26; A61K 8/31; A61K 8/416; A61K 8/585; A61K 8/891;** A61K 2800/31; A61K 2800/42

(86) International application number:
**PCT/JP2023/009185**

(87) International publication number:
**WO 2023/189392 (05.10.2023 Gazette 2023/40)**

(54) **COMPOSITION SUITABLE FOR EYEBROWS**

FÜR AUGENBRAUEN GEEIGNETE ZUSAMMENSETZUNG

COMPOSITION CONVENABLE POUR LES CILS

(84) Designated Contracting States:
**AT BE BG CH CY CZ DE DK EE ES FI FR GB GR HR HU IE IS IT LI LT LU LV MC ME MK MT NL NO PL PT RO RS SE SI SK SM TR**

(30) Priority: **29.03.2022 JP 2022053331**
**29.04.2022 FR 2204052**

(43) Date of publication of application:
**05.02.2025 Bulletin 2025/06**

(73) Proprietor: **L'OREAL**
**75008 Paris (FR)**
Designated Contracting States:
**AT BE BG CH CY CZ DE DK EE ES FI FR GB GR HR HU IE IS IT LI LT LU LV MC ME MK MT NL NO PL PT RO RS SE SI SK SM TR**

(72) Inventor: **OHKUMA Maki**
**Kawasaki-shi**
**Kanagawa 2130012 (JP)**

(74) Representative: **Lavoix**
**Bayerstraße 83**
**80335 München (DE)**

(56) References cited:
**EP-B1- 3 393 428**
**FR-A1- 2 939 034**
**FR-A1- 2 992 203**
**FR-A1- 3 113 598**
**US-A- 6 071 503**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 499 027 B1

## Description

TECHNICAL FIELD

The present invention relates to a composition which is suitable for keratin substances, preferably keratin fibers, and more preferably the eyebrows, as well as a process which relates to the composition.

BACKGROUND OF THE INVENTION

A combination of an MQ resin and a silicone gum has been used with a volatile carrier in anhydrous liquid cosmetic compositions for color transfer resistance or wear resistance. US Patents Nos. 6,074,654, 6,139,823, 6,340,466, 6,406,683, 6,071,503, 6,019,962, 6,464,964, and 7,160,550 relate to this technology. For example, US Patents Nos. 6,074,654 and 6,340,466 disclose a composition for lips, including an MQ resin and a silicone gum in isododecane (cf. Example 6). FR2939034 discloses anhydrous compositions for making-up/ caring of eyelashes/eyebrows comprising a copolymer having silicone resin and silicone fluid, linear/ cyclic volatile silicone oil and nonvolatile linear polydimethylsiloxane.

DISCLOSURE OF INVENTION

For some cosmetic products especially eyebrow makeup products, not only wear resistance but also natural makeup finish such as matte finish are preferable. Natural makeup finish may not be required for compositions for lips such as those disclosed in the above prior art documents. Rather, in many cases, more gloss is required for the composition for lips.

On the other hand, for makeup products, in general, a comfortable texture such as reduced stickiness of the makeup and smooth spreading during application are also preferable. It is especially preferable for liquid makeup products to provide a non-sticky or non-tacky makeup film after being dried.

The addition of powder may contribute to a matte finish or providing a non-sticky makeup film. However, the addition of too much powder ingredients for a matte finish or non-tackiness may worsen smoothness during application.

Furthermore, it is also preferable for liquid makeup products to be stable for a long period of time in particular at elevated temperature.

An objective of the present invention is to provide an anhydrous liquid composition suitable for making up keratin substances, preferably keratin fibers, and more preferably the eyebrows, which is stable and can provide excellent cosmetic effects such as a matte finish, good wear resistance, and comfortable texture such as reduced stickiness of the makeup and smooth spreading during application.

The above objective of the present invention can be achieved by an anhydrous liquid composition for keratin substances, preferably keratin fibers, and more preferably for eyebrows, comprising:

(a) at least one silicone resin;
(b) at least one silicone gum;
(c) at least one volatile hydrocarbon oil;
(d) at least one filler; and
(e) at least one lipophilic gelling agent selected from organomodified clays,

wherein

the (d) filler comprises
(d-1) at least one hydrophobic inorganic filler, preferably selected from hydrophobically-modified metal oxides, and more preferably selected from hydrophobic silicas,
and
wherein
the total amount of the (d) filler and the (e) lipophilic gelling agent in the composition is more than 18.5% by weight, relative to the total weight of the composition,
the amount of the (e) lipophilic gelling agent in the composition is more than 2.5% by weight and less than 10% by weight, relative to the total weight of the composition,
the amount of the (d) filler in the composition is less than 21% by weight, relative to the total weight of the composition, and
the amount of the (d-1) hydrophobic inorganic filler in the composition is less than 2% by weight relative to the total weight of the composition.

The (a) silicone resin may be selected from MQ resins.

The (a) silicone resin may be present in an amount of from 1% to 30% by weight, preferably 5% to 25% by weight, and more preferably 10% to 20% by weight, relative to the total weight of the composition.

The (b) silicone gum may be selected from polydimethylsiloxane gums.

The (b) silicone gum may have a dynamic viscosity at 25 °C of 400,000 $mm^2/s$ or more, preferably 600,000 $mm^2/s$ or more, and more preferably 800,000 $mm^2/s$ or more.

The (b) silicone gum may be present in an amount of from 1% to 25% by weight, preferably 3% to 20% by weight, and more preferably 5% to 15% by weight, relative to the total weight of the composition.

The (c) volatile hydrocarbon oil may be selected from isododecane, C8-9 isoparaffin and a mixture thereof.

The (c) volatile hydrocarbon oil may be present in an amount of from 10% to 60% by weight, preferably from 15% to 55% by weight, and more preferably 20% to 50% by weight, relative to the total weight of the composition.

The (d) filler may be present in an amount of from 10% to 20% by weight, preferably from 12% to 19% by weight and more preferably from 14% to 18% by weight, relative to the total weight of the composition.

The (d) filler may further comprise (d-2) at least one hydrophobic organic filler, preferably selected from hydrophobically-modified starches, and more preferably selected from esterified starches.

The (d-1) hydrophobic inorganic filler may be present in an amount of from 0.1% to less than 2% by weight, preferably from 0.2% to 1.5% by weight, and more preferably from 0.3% to 1% by weight, relative to the total weight of the composition.

The (e) lipophilic gelling agent may be selected from organomodified bentonites, organomodified hectorites, and mixtures thereof.

The (e) lipophilic gelling agent may be present in an amount of from 3% to 9.5% by weight, preferably from 4% to 9% by weight, and more preferably from 5% to 8.5% by weight relative to the total weight of the composition.

The (d) filler and the (e) lipophilic gelling agent may be present in a total amount of from 19% to 35% by weight, preferably from 20% to 30% by weight, and more preferably from 21% to 25% by weight relative to the total weight of the composition.

The present invention may also relate to a cosmetic process for keratin substances, preferably keratin fibers, and more preferably the eyebrows, comprising the steps of:
applying the composition according to the present invention onto the keratin substances.

## DETAILED DESCRIPTION OF THE INVENTION

After diligent research, the inventors have discovered that it is possible to provide an anhydrous liquid composition suitable for making up keratin substances, preferably keratin fibers, and more preferably the eyebrows, which is stable and can provide excellent cosmetic effects such as a matte finish, good wear resistance, and a comfortable texture such as reduced stickiness of the makeup and smooth spreading during application.

Thus, the composition according to the present invention is an anhydrous liquid composition for keratin substances, preferably keratin fibers, and more preferably the eyebrows, comprising:

(a) at least one silicone resin;
(b) at least one silicone gum;
(c) at least one volatile hydrocarbon oil;
(d) at least one filler; and
(e) at least one lipophilic gelling agent selected from organomodified clays,

wherein

the (d) filler comprises
(d-1) at least one hydrophobic inorganic filler, preferably selected from hydrophobically-modified metal oxides, and more preferably selected from hydrophobic silicas,
and
wherein
the total amount of the (d) filler and the (e) lipophilic gelling agent in the composition is more than 18.5% by weight, relative to the total weight of the composition,
the amount of the (e) lipophilic gelling agent in the composition is more than 2.5% by weight and less than 10% by weight, relative to the total weight of the composition,
the amount of the (d) filler in the composition is less than 21% by weight, relative to the total weight of the composition, and
the amount of the (d-1) hydrophobic inorganic filler in the composition is less than 2% by weight relative to the total

weight of the composition.

The (d) filler is different from the (e) lipophilic gelling agent.

The composition according to the present invention is stable and can provide excellent cosmetic effects such as a matte finish, good wear resistance, and a comfortable texture such as reduced stickiness of the makeup and smooth spreading during application.

The composition according to the present invention is stable for a long period of time, even at elevated temperatures. Therefore, the composition according to the present invention can be stored for a long period of time, even under hot conditions.

The composition according to the present invention can provide keratin substances, preferably keratin fibers, and more preferably the eyebrows, with a matte finish or a less glossy finish. Namely, the composition according to the present invention can provide a natural makeup finish.

The composition according to the present invention can provide a comfortable texture such as reduced stickiness of the makeup and smooth spreading during application. Namely, the composition according to the present invention can smoothly slide or glide on keratin substances, preferably keratin fibers, and more preferably the eyebrows.

The cosmetic effects provided by the composition according to the present invention can be long-lasting or can be maintained for a long period of time. The cosmetic effects provided by the composition according to the present invention can have good resistance to sebum or water. Therefore, the makeup by the composition according to the present invention can last for a long period of time even when exposed to, for example, sweat or rain. Thus, the composition according to the present invention can also provide keratin substances, preferably keratin fibers, and more preferably the eyebrows, with good wear resistance.

The composition according to the present invention can also provide makeup with color transfer resistance or wear resistance.

Hereinafter, the composition and process according to the present invention will be explained in a more detailed manner.

[Composition]

(Silicone Resin)

The composition according to the present invention comprises (a) at least one silicone resin. If two or more silicone resins are used, they may be the same or different.

The term "resin" means a compound whose structure is three-dimensional. "Silicone resins" are also known as "silicone-based resins" or "siloxane resins". Thus, for the purposes of the present invention, a linear polydimethylsiloxane is not a silicone resin.

The nomenclature of silicone resins (also known as siloxane resins) is known under the name "MDTQ", with the resin being described as a function of the various siloxane monomer units that it comprises, where each of the letters "MDTQ" characterize a type of unit.

The letter "M" represents the Monofunctional unit of formula $R_1R_2R_3SiO_{1/2}$, in which the silicon atom is connected to only one oxygen atom in the polymer comprising this unit.

The letter "D" means a difunctional unit $R_1R_2SiO_{2/2}$ in which the silicon atom is connected to two oxygen atoms.

The letter "T" represents a Trifunctional unit of formula $R_1SiO_{3/2}$.

Such resins are described, for example, in the "Encyclopedia of Polymer Science and Engineering, vol. 15, John Wiley & Sons, New York (1989), pp. 265-270 and USP 2,676,182, USP 3,627,851, USP 3,772,247 and USP 5,248,739 or alternatively USP 5,082,706, USP 5,319,040, USP 5,302,685 and USP 4,935,484.

In the M, D and T units defined previously, R, i.e., $R_1$, $R_2$, and $R_3$, represents a hydrocarbon-based radical (especially alkyl) containing from 1 to 10 carbon atoms, a phenyl group, a phenylalkyl group or a hydroxyl group.

Finally, the letter "Q" means a tetrafunctional unit $SiO_{4/2}$ in which the silicon atom is linked to four oxygen atoms, which are themselves linked to the rest of the polymer.

Various silicone resins with different properties may be obtained from these different units, the properties of these polymers varying as a function of the type of monomer (or unit), the nature and number of the radical R, the length of the polymer chain, the degree of branching and the size of the pendent chains.

As silicone resins that may be used in the composition according to the present invention, use may be made, for example, of silicone resins of MQ type, of T type or of MQT type.

MQ Resins:

As examples of silicone resins of MQ type, mention may be made of the alkyl siloxysilicates of formula

$[(R1)_3SiO_{1/2}]_x(SiO_{4/2})_y$ (MQ units) in which x and y are integers ranging from 50 to 80, and such that the group R1 represents a radical as defined previously, and is preferably an alkyl group containing from 1 to 8 carbon atoms, preferably a methyl group.

As examples of solid silicone resins of MQ type of trimethyl siloxysilicate type, mention may be made of those sold under the reference SR1000 by General Electric Co., under the reference TMS 803 by Wacker Chemie AG, or under the name KF-7312J by Shin-Etsu Chemical Co., Ltd. or DC749 or DC593 by Dow Corning Corporation.

As silicone resins comprising MQ siloxysilicate units, mention may also be made of phenylalkylsiloxysilicate resins, such as phenylpropyldimethylsiloxysilicate (Silshine 151 sold by the company General Electric). The preparation of such resins is described especially in patent USP 5,817,302.

Resins T:

Examples of these silicone resins of type T that may be mentioned include the polysilsesquioxanes of formula $(RSiO_{3/2})_x$ (units T) in which x is greater than 100 and such that the group R is an alkyl group containing from 1 to 10 carbon atoms, the said polysilsesquioxanes also possibly comprising Si-OH end groups.

Polymethylsilsesquioxane resins that may preferably be used are those in which R represents a methyl group, for instance those sold:

- by the company Wacker under the reference Resin MK, such as Belsil PMS MK: polymer comprising $CH_3SiO_{3/2}$ repeating units (units T), which may also comprise up to 1% by weight of $(CH_3)_2SiO_{2/2}$ units (units D) and having an average molecular weight of about 10 000 g/mol, or
- by the company Shin-Etsu under the reference KR-220L, which are composed of units T of formula $CH_3SiO_{3/2}$ and have Si-OH (silanol) end groups, under the reference KR-242A, which comprise 98% of units T and 2% of dimethyl units D and have Si-OH end groups, or alternatively under the reference KR-251 comprising 88% of units T and 12% of dimethyl units D and have Si-OH end groups.

MQT Resins:

Resins comprising MQT units that are especially known are those mentioned in document USP 5,110,890.

A preferred form of resins of MQT type are MQT-propyl (also known as MQTPr) resins. Such resins that may be used in the composition according to the present invention are the resins described and prepared in patent application WO 2005/075 542, the content of which is incorporated herein by reference.

The MQ-T-propyl resin preferably comprises the following units:

(i) $(R1_3SiO_{1/2})_a$
(ii) $(R2_2SiO_{2/2})_b$
(iii) $(R3SiO_{3/2})_c$ and
(iv) $(SiO_{4/2})_d$

with

R1, R2 and R3 independently representing a hydrocarbon-based radical (especially alkyl) containing from 1 to 10 carbon atoms, a phenyl group, a phenylalkyl group or a hydroxyl group, and preferably an alkyl radical containing from 1 to 8 carbon atoms or a phenyl group, a being between 0.05 and 0.5,
b being between 0 and 0.3,
c being greater than 0,
d being between 0.05 and 0.6,
a + b + c + d = 1, and a, b, c and d being mole fractions,
on the condition that more than 40 mol% of the groups R3 of the siloxane resin are propyl groups.

Preferably, the siloxane resin comprises the following units:

(i) $(R1_3SiO_{1/2})_a$
(iii) $(R3SiO_{3/2})_c$ and
(iv) $(SiO_{4/2})_d$

with

R1 and R3 independently representing an alkyl group containing from 1 to 8 carbon atoms,
R1 preferably being a methyl group and R3 preferably being a propyl group,
a being between 0.05 and 0.5 and preferably between 0.15 and 0.4,
c being greater than 0 and preferably between 0.15 and 0.4,
d being between 0.05 and 0.6, preferably between 0.2 and 0.6, or alternatively between 0.2 and 0.55,
a + b + c + d = 1 and a, b, c and d being mole fractions,
on the condition that more than 40 mol% of the groups R3 of the siloxane resin are propyl groups.

The siloxane resins that may be used according to the present invention may be obtained via a process comprising the reaction of:

A) an MQ resin comprising at least 80 mol% of units $(R1_3SiO_{1/2})_a$ and $(SiO_{4/2})_d$

R1 representing an alkyl group containing from 1 to 8 carbon atoms, an aryl group, a carbinol group or an amino group,
a and d being greater than 0,
the ratio a/d being between 0.5 and 1.5;
and

B) a T-propyl resin comprising at least 80 mol% of units $(R3SiO_{3/2})_c$,

R3 representing an alkyl group containing from 1 to 8 carbon atoms, an aryl group, a carbinol group or an amino group,
c being greater than 0,
on the condition that at least 40 mol% of the groups R3 are propyl groups,
in which the mass ratio A/B is between 95/5 and 15/85 and the mass ratio A/B is preferably 30/70.

Advantageously, the mass ratio A/B is between 95/5 and 15/85. Preferably, the ratio A/B is less than or equal to 70/30. These preferred ratios have been proven to produce deposits that are comfortable due to the absence of percolation of the rigid MQ resin particles in the deposit.
Thus, preferably, the silicone resin is chosen from the group comprising:

a) a resin of MQ type, chosen especially from (i) alkyl siloxysilicates, which may be trimethyl siloxysilicates, of formula $[(R1)_3SiO_{1/2}]_x(SiO_{4/2})_y$, in which x and y are integers ranging from 50 to 80, and such that the group R1 represents a hydrocarbon-based radical containing from 1 to 10 carbon atoms, a phenyl group, a phenylalkyl group or a hydroxyl group, and preferably is an alkyl group containing from 1 to 8 carbon atoms, preferably a methyl group, and (ii) phenylalkyl siloxysilicate resins, such as phenylpropyldimethyl siloxysilicate, and/or
b) a resin of T type, chosen especially from the polysilsesquioxanes of formula $(RSiO_{3/2})_x$, in which x is greater than 100 and the group R is an alkyl group containing from 1 to 10 carbon atoms, for example a methyl group, the said polysilsesquioxanes also possibly comprising Si-OH end groups, and/or
c) a resin of MQT type, especially of MQT-propyl type, which may comprise units (i) $(R1_3SiO_{1/2})_a$, (ii) $(R2_2SiO_{2/2})_b$, (iii) $(R3SiO_{3/2})_c$ and (iv) $(SiO_{4/2})_d$,

with R1, R2 and R3 independently representing a hydrocarbon-based radical, especially alkyl, containing from 1 to 10 carbon atoms, a phenyl group, a phenylalkyl group or a hydroxyl group and preferably an alkyl radical containing from 1 to 8 carbon atoms or a phenyl group, a being between 0.05 and 0.5,
b being between 0 and 0.3,
c being greater than 0,
d being between 0.05 and 0.6,
a + b + c + d = 1, and a, b, c and d being mole fractions,
on the condition that more than 40 mol% of the groups R3 of the siloxane resin are propyl groups.

It is preferable that the (a) silicone resin be selected from MQ resins, and more preferably be trimethylsiloxysilicate.
The amount of the (a) silicone resin in the composition according to the present invention may be 1% by weight or more, preferably 5% by weight or more, and more preferably 10% by weight or more, relative to the total weight of the composition.
The amount of the (a) silicone resin in the composition according to the present invention may be 30% by weight

or less, preferably 25% by weight or less, and more preferably 20% by weight or less, relative to the total weight of the composition.

Thus, the amount of the (a) silicone resin in the composition according to the present invention may be from 1% to 30% by weight, preferably from 5% to 25% by weight, and more preferably from 10% to 20% by weight, relative to the total weight of the composition.

(Silicone Gum)

The composition according to the present invention comprises (b) at least one silicone gum. If two or more (b) silicone gums are used, they may be the same or different.

The (b) silicone gum may have a dynamic viscosity at 25 °C of 400,000 $mm^2/s$ or more, preferably 600,000 $mm^2/s$ or more, and more preferably 800,000 $mm^2/s$ or more.

The (b) silicone gum may have a dynamic viscosity at 25 °C of 10,000,000 $mm^2/s$ or less, preferably 5,000,000 $mm^2/s$ or less, and more preferably 3,000,000 $mm^2/s$ or less.

The (b) silicone gum may have a dynamic viscosity at 25 °C of from 300,000 to 10,000,000 $mm^2/s$, preferably from 500,000 to 5,000,000 $mm^2/s$ or less, and more preferably from 700,000 to 3,000,000 $mm^2/s$.

The dynamic viscosity of the (b) silicone gum can be measured according to standard ASTM D-445.

It may be preferable that the (b) silicone gum has no functional group such as an amino group.

The (b) silicone gum may be chosen especially from the silicones of formula:

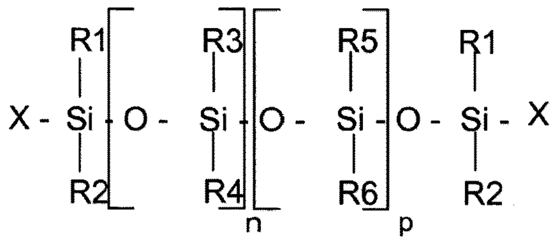

in which:

R1, R2, R5 and R6 are, together or separately, an alkyl radical containing 1 to 6 carbon atoms, R3 and R4 are, together or separately, an alkyl radical containing from 1 to 6 carbon atoms, a vinyl radical, an amine radical or a hydroxyl radical,
X is an alkyl radical containing from 1 to 6 carbon atoms, a hydroxyl radical or an amine radical, and
n and p being integers chosen such that the dynamic viscosity at 25 °C of the compound is 400,000 $mm^2/s$ or more, preferably 600,000 $mm^2/s$ or more, and more preferably 800,000 $mm^2/s$ or more.

As silicone gums that may be used according to the present invention, mention may be made of those for which:

- the substituents R1 to R6 represent a methyl group, the group X represents a methoxy group, and n and p are such that the molecular weight of the polymer is 600 000 g/mol, such as the product sold under the name Mirasil C-DPDM by the company Bluestar;
- the substituents R1 to R6 represent a methyl group, the group X represents a hydroxyl group, and n and p are such that the molecular weight of the polymer is 600 000 g/mol, such as the product sold under the name SGM 36 by the company Dow Corning; and
- dimethicones of the (polydimethylsiloxane) (methylvinylsiloxane) type, such as SE63 sold by GE Bayer Silicones, poly(dimethylsiloxane)(diphenyl)(methylvinylsiloxane) copolymers, and mixtures thereof.

The molecular mass of the (b) silicone gum may be greater than 350,000 g/mol, between 350,000 and 800,000 g/mol, and preferably from 450,000 to 700,000 g/mol.

It is preferable that the (b) silicone gum be selected from polydimethylsiloxane gum.

The amount of the (b) silicone gum in the composition according to the present invention is 1% by weight or more, and may be preferably 3% by weight or more, and more preferably 5% by weight or more, relative to the total weight of the composition.

The amount of the (b) silicone gum in the composition according to the present invention is 25% by weight or less, and may be preferably 20% by weight or less, and more preferably 15% by weight or less, relative to the total weight of the composition.

Thus, the amount of the (b) silicone gum in the composition according to the present invention is from 1% to 25% by weight, preferably from 3% to 20% by weight, and more preferably from 5% to 15% by weight, relative to the total weight of the composition.

(Volatile Hydrocarbon Oil)

The composition according to the present invention comprises (c) at least one volatile hydrocarbon oil. If two or more (c) volatile hydrocarbon oils are used, they may be the same or different.
Here, "oil" means a fatty compound or substance which is in the form of a liquid or a paste (non-solid) at room temperature (25°C) under atmospheric pressure (760 mmHg).
The term "volatile oil" is understood to mean, within the meaning of the present invention, an oil which is capable of evaporating on contact with the skin or the keratinous fibre in less than one hour, at ambient temperature (20-25 °C) and atmospheric pressure. The volatile oils of the present invention are volatile cosmetic oils which are liquid at ambient temperature with a non-zero vapour pressure, at ambient temperature and atmospheric pressure, ranging in particular from 0.13 Pa to 40 000 Pa ($10^{-3}$ to300 mmHg), in particular ranging from 1.3 Pa to 13 000 Pa (0.01 to 100 mmHg) and more particularly ranging from 1.3 Pa to 1300 Pa (0.01 to 10 mmHg).
Mention may be made, as examples of volatile hydrocarbon oil which can be used in the present invention, of volatile hydrocarbon oils chosen from hydrocarbon oils having from 8 to 16 carbon atoms, in particular $C_8$-$C_{16}$ isoalkanes of petroleum origin (also known as isoparaffins), such as isododecane (also known as 2,2,4,4,6-pentamethylheptane), isodecane or isohexadecane, for example the oils sold under the Isopar or Permethyl trade names, branched $C_8$-$C_{16}$ esters, isohexyl neopentanoate and their mixtures. Use may also be made of other volatile hydrocarbon oils, such as petroleum distillates, in particular those sold under the name Shell Solt by Shell; volatile linear alkanes, such as those described in Patent Application DE10 2008 012 457 from Cognis.
Preference is given, among volatile hydrocarbon oils, to $C_8$-$C_{16}$ isoalkanes, in particular isododecane.
It is preferable that the (c) volatile hydrocarbon oil be chosen from isododecane, C8-9 isoparaffin and a mixture thereof.
The amount of the (c) volatile hydrocarbon oil(s) in the composition according to the present invention may be 10% by weight or more, preferably 15% by weight or more, and more preferably 20% by weight or more, relative to the total weight of the composition.
The amount of the (c) volatile hydrocarbon oil(s) in the composition according to the present invention may be 60% by weight or less, preferably 55% by weight or less, and more preferably 50% by weight or less, relative to the total weight of the composition.
The amount of the (c) volatile hydrocarbon oil(s) in the composition according to the present invention may be from 10% to 60% by weight, preferably from 15% to 55% by weight, and more preferably from 20% to 50% by weight, relative to the total weight of the composition.

(Filler)

The composition according to the present invention comprises (d) at least one filler. If two or more (d) fillers are used, they may be the same or different.
The term "filler" should be understood as meaning a colorless or white, inorganic or synthetic particle which is insoluble in a possible liquid component in the composition according to the present invention, whatever the temperature at which the composition is manufactured.
The (d) filler(s) can be inorganic or organic, and can be of spherical or oblong shape, whatever the crystallographic form (for example, sheet, cubic, hexagonal, orthorhombic, and the like). Non-limiting mention may be made of talc, mica, silica, silica silylate, kaolin, sericite, calcinated talc, calcinated mica, calcinated sericite, synthetic mica, bismuth oxychloride, barium sulfate, boron nitride, calcium carbonate, magnesium carbonate, magnesium hydrogen carbonate and hydroxyapatite, powders formed of polyamide (Nylon®), of poly-β-alanine and of polyethylene, powders formed of polyurethane, powders formed of tetrafluoroethylene polymers (Teflon®), lauryllysine, starch, polymeric hollow microspheres, such as those of poly(vinylidene chloride)/acrylonitrile, for example Expancel® (Nobel Industrie), or of acrylic acid copolymers, silicone resin microbeads (Tospearls® from Toshiba, for example), particles formed of polyorganosiloxane elastomers, precipitated calcium carbonate, magnesium carbonate, basic magnesium carbonate, hollow silica microspheres, glass or ceramic microcapsules, or metal soaps derived from organic carboxylic acids having from 8 to 22 carbon atoms, such as from 12 to 18 carbon atoms, for example zinc stearate, magnesium stearate, lithium stearate, zinc laurate or magnesium myristate.
For the present invention, examples of the (d) filler include metal oxides, preferably silica, titanium oxide, zinc oxide and a mixture thereof.
A filler that is suitable for the present invention may be, for example, a filler whose mean particle size is less than

100 μm, and especially between 1 and 50 μm, for example between 4 to 20 μm.

The (d) filler may be hydrophilic or hydrophobic.

The (d) filler may be or may not be capable of absorbing (and/or adsorbing) an oil or a liquid fatty substance, for instance sebum (from the skin). It is preferable that the (d) filler be capable of absorbing (and/or adsorbing) an oil or a liquid fatty substance, for instance sebum (from the skin).

The hydrophilic or hydrophobic oil-absorbing powder may comprise porous or hollow particles, in particular porous or hollow spherical particles.

Hydrophilic Oil-Absorbing Powder:

For the purpose of the present invention, the term "hydrophilic" oil-absorbing powder means that said powder (or the particles) can be individually dispersed in water in such a manner that aggregates are not formed.

The hydrophilic oil-absorbing powder may have an oil-absorbing capacity of 100 ml/100 g or more, preferably 150 ml/100 g or more, and more preferably 200 ml/100 g or more.

The amount of oil absorbed (and/or adsorbed) by the hydrophilic oil-absorbing powder may be characterized by measuring the wet point according to the method described below. The oil-absorbing capacity measured at the wet point, noted Wp, corresponds to the amount of oil that needs to be added to 100 g of powder in order to obtain a homogeneous paste.

The amount of the absorbed (and/or adsorbed) oil can be measured according to the method for determining the oil uptake of a powder described in standard NF T 30-022. It corresponds to the amount of oil absorbed/adsorbed onto the available surface of the powder, by measuring the wet point.

An amount of m = 2 g of powder is placed on a glass plate, and an oil (such as ester oil and silicone oil) is then added drop-wise. After addition of 4 to 5 drops of oil to the powder, mixing is performed using a spatula, and addition of oil is continued until a conglomerate of oil and powder has formed. At this point, the oil is added one drop at a time and the mixture is then triturated with the spatula. The addition of oil is stopped when a firm, smooth paste is obtained. This paste must be able to be spread on the glass plate without cracking or forming lumps. The volume Vs (expressed in ml) of oil used is then noted. The oil uptake corresponds to the ratio Vs/m.

Otherwise, the oil-absorbing capacity can be measured in accordance with JIS-K6217-4.

The hydrophilic oil-absorbing powder may be of organic or inorganic nature.

The hydrophilic oil-absorbing powder may be chosen from celluloses, silicas, silicates; perlites; magnesium carbonate; magnesium hydroxide; and derivatives thereof; and a mixture thereof.

According to one embodiment, a cellulose derivative may be chosen from cellulose esters and ethers.

The term "cellulose ester" means, in the text hereinabove and hereinbelow, a polymer consisting of an α (1-4) sequence of partially or totally esterified anhydroglucose rings, the esterification being obtained by reaction of all or only some of the free hydroxyl functions of the said anhydroglucose rings with a linear or branched carboxylic acid or carboxylic acid derivative (acid chloride or acid anhydride) containing from 1 to 4 carbon atoms.

Preferably, the cellulose ester results from the reaction of some of the free hydroxyl functions of the said rings with a carboxylic acid containing from 1 to 4 carbon atoms. Advantageously, the cellulose esters are chosen from cellulose acetates, propionates, butyrates, isobutyrates, acetobutyrates and acetopropionates, and mixtures thereof.

The term "cellulose ether" means a polymer consisting of an α (1-4) sequence of partially etherified anhydroglucose rings, some of the free hydroxyl functions of the said rings being substituted with a radical -OR, R preferably being a linear or branched alkyl radical containing from 1 to 4 carbon atoms. The cellulose ethers are thus preferably chosen from cellulose alkyl ethers with an alkyl group containing from 1 to 4 carbon atoms, such as cellulose methyl, propyl, isopropyl, butyl and isobutyl ethers.

Celluloses and their derivatives that may be mentioned include, for example, the following spherical cellulose particles marketed by Daito Kasei in Japan: Cellulobeads USF (oil uptake is 250 ml/100 g) with a particle size of 4 μm (porous cellulose).

Silica powders that may be mentioned include porous silica microspheres, especially those sold under the names Sunsphere® H31 and Sunsphere® H51 (oil uptake equal to 150 ml/100 g) by the company Asahi Glass; MSS-500-3H by the company Kobo; amorphous hollow silica particles, especially those sold under the name Silica Shells by the company Kobo (oil uptake equal to 550 ml/100 g); porous silica microsphere sold under the name of Silysia 350 (oil uptake equal to 310 ml/100 g) by the company Fuji Silysia Chemical; and silica powder sold under the name of Finesil X35 (oil uptake equal to 380 ml/100 g)by the company Oriental Silycas.

A silicate that may especially be mentioned is aluminum silicate which is sold under the name of Kyowaad® 700PEL (oil uptake equal to 195 ml/100 g) by the company Kyowa Chemical Industry.

A perlite powder that may especially be mentioned is the product sold under the name Optimat® 1430 OR and Optimat® 2550 OR by the company World Minerals (oil uptake equal to 240 ml/100 g).

A magnesium carbonate powder that may especially be mentioned is the product sold under the name Tipo

Carbomagel® by the company Buschle & Lepper (oil uptake equal to 214 ml/100 g).

A magnesium carbonate/magnesium hydroxide powder that may especially be mentioned is the product of $mMgCO_3$-$Mg(OH)_2$-$nH_2O$ which is sold under the name of Mg Tube (oil uptake equal to 250-310 ml/100 g) by the company Nittetsu Mining.

It is preferable that the hydrophilic oil-absorbing powder comprise at least one selected from the group consisting of cellulose, silica, perlite, and a mixture thereof.

Hydrophobic Oil-Absorbing Powder:

For the purpose of the present invention, the term "hydrophobic" oil-absorbing powder means that said powder (or the particles) can be individually dispersed in an oil in such a manner that aggregates are not formed.

The hydrophobic oil-absorbing powder may have an oil-absorbing capacity of 100 ml/100 g or more, preferably 150 ml/100 g or more, and more preferably 200 ml/100 g or more.

The amount of oil absorbed (and/or adsorbed) by the hydrophobic oil-absorbing powder may be determined by the method described above.

The hydrophobic oil-absorbing powder may be of organic or inorganic nature.

Organic Hydrophobic Oil-Absorbing Powder;

The organic hydrophobic oil-absorbing powder may be chosen from the group consisting of polyamide (in particular Nylon-6) powders, powders of acrylic polymers, especially of polymethyl methacrylate, of polymethyl methacrylate/ethylene glycol dimethacrylate, of polyallyl methacrylate/ethylene glycol dimethacrylate or of ethylene glycol dimethacrylate/lauryl methacrylate copolymer; and a mixture thereof. The above material may be crosslinked.

It may be preferable that the organic hydrophobic oil-absorbing powder be selected from powders of acrylic polymers, especially of ethylene glycol dimethacrylate/lauryl methacrylate copolymer.

Examples of the organic hydrophobic oil-absorbing powder include the fillers described below.

Acrylic polymer powders that may be mentioned include porous polymethyl methacrylate (INCI name methyl methacrylate crosspolymer) such as the spheres sold under the name Covabead LH85 by the company Sensient, porous polymethyl methacrylate/ethylene glycol dimethacrylate spheres sold under the name Microsponge 5640 by the company Cardinal Health Technologies (oil uptake equal to 155 ml/100 g), ethylene glycol dimethacrylate/lauryl methacrylate crosslinked copolymer powders, especially those sold under the name Polytrap® 6603 from the company Amcol Health & Beauty Solutions (oil uptake equal to 656 ml/100 g), acrylonitrile/methyl methacrylate/vinylidene chloride copolymer sold under the name Expancel 551DE40D42 (oil uptake equal to 1,040 ml/100 g) by the company Akzo Novel.

Polyamide powders that may be mentioned include Nylon-6 powder, especially the product sold under the name Pomp610 by the company UBE Industries (oil uptake equal to 202 ml/100 g).

Inorganic Hydrophobic Oil-Absorbing Powder;

The inorganic hydrophobic oil-absorbing powder may have at least one inorganic core and at least one hydrophobic coating.

It is preferable that the inorganic hydrophobic oil-absorbing powder be selected from powders of hydrophobic silicas, preferably hydrophobic silica aerogels, and more preferably hydrophobic aerogels of silica silylate, and a mixture thereof.

The term "hydrophobic silica" is understood to mean any silica, the surface of which is treated to be hydrophobic.

The hydrophobic silica, in particular silica silylate, may be based on silica aerogels which are porous materials obtained by replacing (by drying) the liquid component of a silica gel with air.

They are generally synthesized via a sol-gel process in a liquid medium and then dried, usually by extraction with a supercritical fluid, the one most commonly used being supercritical $CO_2$. This type of drying makes it possible to avoid shrinkage of the pores and of the material. The sol-gel process and the various drying operations are described in detail in Brinker C.J. and Scherer G.W., Sol-Gel Science, New York, Academic Press, 1990.

Aerogels are materials with high porosity. Herein, silica aerogels refer to a solid silica with a porous structure generally obtained by replacing medium included in wet silica gels with air by drying them while a solid network structure of the silica is maintained. The porosity represents the amount of air contained in an apparent volume of a material by a volume percentage. The hydrophobic silica aerogel of the present invention may have a porosity of 60% or more, preferably 70% or more, and more preferably 80% or more.

The hydrophobic silica aerogel particles may exhibit

a specific surface area per unit of weight (SW) ranging from 500 to 1,500 $m^2/g$, preferably from 600 to 1,200 $m^2/g$, and

more preferably from 600 to 800 $m^2/g$, and/or

a size, expressed as the volume-average diameter (D[0.5]), ranging from 1 to 1,500 μm, preferably from 1 to 1,000 μm, more preferably from 1 to 100 μm, in particular from 1 to 30 μm, more preferably from 5 to 25 μm, more preferably from 5 to 20 μm, and even more preferably from 5 to 15 μm.

The specific surface area per unit of weight can be determined by the nitrogen absorption method, known as the BET (Brunauer-Emmett-Teller) method, described in The Journal of the American Chemical Society, Vol. 60, page 309, February 1938, which corresponds to international standard ISO 5794/1 (appendix D). The BET specific surface area corresponds to the total specific surface area of the particles under consideration.

The sizes of the hydrophobic silica aerogel particles can be measured by static light scattering using a commercial particle size analyzer of MasterSizer 2000 type from Malvern. The data are processed on the basis of the Mie scattering theory. This theory, which is exact for isotropic particles, makes it possible to determine, in the case of non-spherical particles, an "effective" particle diameter. This theory is described in particular in the publication by Van de Hulst, H.C., "Light Scattering by Small Particles", Chapters 9 and 10, Wiley, New York, 1957.

The hydrophobic silica aerogel particles can advantageously exhibit a packed density (r) ranging from 0.04 $g/cm^3$ to 0.10 $g/cm^3$, and preferably from 0.05 $g/cm^3$ to 0.08 $g/cm^3$.

In the context of the present invention, this density, known as the packed density, can be assessed according to the following protocol:

40 g of powder are poured into a graduated measuring cylinder;

the measuring cylinder is then placed on the Stav 2003 device from Stampf Volumeter;

the measuring cylinder is subsequently subjected to a series of 2500 packing actions (this operation is repeated until the difference in volume between 2 consecutive tests is less than 2%); and

the final volume Vf of packed powder is then measured directly on the measuring cylinder. The packed density is determined by the ratio w/Vf, in this instance 40/Vf (Vf being expressed in $cm^3$ and w in g).

Regarding the preparation of hydrophobic silica aerogel particles modified at the surface by silylation, reference may be made to the document US 7 470 725.

Use will in particular be made of hydrophobic silica aerogel particles modified at the surface with trimethylsilyl groups.

The inorganic hydrophobic oil-absorbing powders that may be mentioned include polydimethylsiloxane-coated amorphous silica microspheres, especially those sold under the name Sunsphere® H33 and Sunsphere® H53 (oil uptake equal to 400 ml/100 g), precipitated silica powders surface-treated with a mineral wax, such as precipitated silica treated with a polyethylene wax, and especially those sold under the name Acematt OR 412 by the company Evonik-Degussa (oil uptake equal to 398 ml/100 g), and silica silylate sold under the name of VM-2270 (oil uptake equal to 1,040 ml/100 g) by the company Dow.

It is preferable to use, as the inorganic hydrophobic oil-absorbing powder, silica silylate sold under the name VM-2270 by Dow, the particles of which exhibit an average size ranging from 5 to 15 μm and a specific surface area per unit of weight ranging from 600 to 800 $m^2/g$.

The hydrophobic silica aerogel particles may be characterized in that the shape of each of the particles is spherical. Due to this spherical shape, the hydrophobic silica aerogel particles can provide cosmetic compositions with good smoothness. The spherical degree of the hydrophobic silica aerogel may be determined by an average circularity.

The spherical hydrophobic silica aerogel particle may have an average circularity of 0.8 or more, and preferably 0.82 or more. The spherical hydrophobic silica aerogel may have an average circularity of less than 1, preferably 0.99 or less, more preferably 0.98 or less, even more preferably 0.97 or less, still even more preferably 0.96 or less, and most preferably 0.95 or less.

The "average circularity" may be determined by an image analysis method. In particular, the "average circularity" may be an arithmetic mean of circularity obtained by image analysis of a scanning electron microscope (SEM) image of no less than 2,000 aerogel particles observed at a magnification of 1,000 by secondary electron detection using a scanning electron microscope (SEM).

The "circularity" of each aerogel particle is a value determined by the following formula:

$$C = 4\pi S / L^2$$

wherein C represents circularity, S represents the area (projected area) of the aerogel particle in the image, and L represents the length of a periphery (perimeter) of the aerogel particle in the image. When the average circularity approaches 1, the shape of each of the particles becomes more spherical.

The hydrophobic silica aerogel particles that may be used according to the present invention is preferably of

silylated silica type (INCI name: silica silylate). Preferably, the hydrophobic silica aerogel particles may be those described in JP-A-2014-088307, JP-A-2014-218433, or JP-A-2018-177620.

It is preferable to use hydrophobic aerogels of silica silylate as the inorganic hydrophobic oil-absorbing powder.

The hydrophobicity of the hydrophobic aerogels of silica silylate may be obtained by reacting a hydrophobicizing agent with a silanol group represented by the following formula existing on the surface of silica:

$$\equiv Si\text{-}OH$$

wherein the symbol "≡" represents the remaining three valences of the Si atom, thereby converting the silanol group into a group represented by the following formula:

$$(\equiv Si\text{-}O\text{-})_{(4-n)}SiR_n$$

wherein n is an integer of 1 to 3; each R is independently a hydrocarbyl group; and two or more R may be the same or different from each other where n is 2 or more.

The hydrophobicizing agent may be a silylating agent. Therefore, according to one preferred embodiment, in the hydrophobic aerogels of silica silylate, the silica particles may be modified at the surface by silylation. As examples of the silylating agents, mention may be made of a treating agent having one of the following formulae (1) to (3).

Formula (1): $R_nSiX_{(4-n)}$

wherein n represents an integer of 1 to 3; R represents a hydrocarbyl group; X represents a group (i.e. a leaving group) which can leave a molecule by cleavage of the bond with the Si atom in a reaction with a compound having a hydroxyl group; each R may be different where n is 2 or more; and each X may be different where n is 2 or less.

Formula (2):

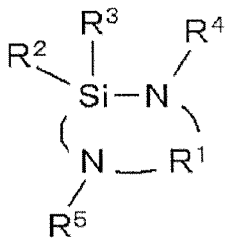

wherein $R^1$ represents an alkylene group; $R^2$ and $R^3$ independently represent a hydrocarbyl group; and $R^4$ and $R^5$ independently represent a hydrogen atom or a hydrocarbyl group.

Formula (3):

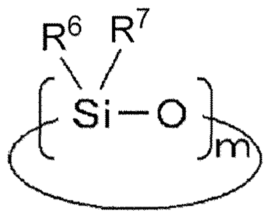

wherein $R^6$ and $R^7$ independently represent a hydrocarbyl group; m represents an integer of 3 to 6; each $R^6$ may be different when there are two or more $R^6$; and each $R^7$ may be different when there are two or more $R^7$.

In the above formula (1), R is a hydrocarbyl group, preferably a hydrocarbyl group having a carbon number of 1 to 10, more preferably a hydrocarbyl group having a carbon number of 1 to 4, and especially preferably a methyl group.

As examples of the leaving group represented by X, mention may be made of halogen atoms such as chlorine and bromine; alkoxy groups such as methoxy group and ethoxy group; groups represented by $-NH-SiR_3$ (wherein the definition of R is the same as that of R in formula (1)).

Specific examples of the hydrophobicizing agent represented by the above formula (1) include: chlorotrimethylsilane, dichlorodimethylsilane, trichloromethylsilane, monomethyltrimethoxysilane, monomethyltriethoxysilane, and hexamethyldisilazane.

Most preferably, chlorotrimethylsilane, dichlorodimethylsilane, trichloromethylsilane, and/or hexamethyldisilazane may be used from the viewpoint of favorable reactivity.

The number of bonds of the Si atom with the silanol group on the silica framework varies depending on the number (4-n) of the leaving group X. For example, if n is 2, the following bonding will occur:

$$(\equiv Si\text{-}O\text{-})_2SiR_2.$$

If n is 3, the following bonding will occur:

$$\equiv Si\text{-}O\text{-}SiR_3.$$

In this manner, the silanol groups may be silylated, and thereby hydrophobization may be carried out.

In the above formula (2), $R^1$ may be an alkylene group, preferably an alkylene group having a carbon number of 2 to 8, and especially preferably an alkylene group having a carbon number of 2 to 3.

In the above formula (2), $R^2$ and $R^3$ are independently a hydrocarbyl group, and the same preferable groups as those of R in the formula (1) can be mentioned. $R^4$ represents a hydrogen atom or a hydrocarbyl group, and when it is a hydrocarbyl group, the same preferable groups as those of R in the formula (1) can be mentioned. When a gel of silica is treated with the compound (cyclic silazane) represented by formula (2), cleavage of Si-N bonds will occur by the reaction with silanol groups, and therefore the following bonding will occur on the surface of the silica framework in the gel:

$$(\equiv Si\text{-}O\text{-})_2SiR^2R^3.$$

In this way, the silanol group may be silylated by the cyclic silazanes of the above formula (2) as well, and thereby hydrophobization may be carried out.

Specific examples of the cyclic silazanes represented by the above formula (3) include hexamethylcyclotrisilazane, and octamethylcyclotetrasilazane.

In the above formula (3), $R^6$ and $R^7$ are independently a hydrocarbyl group, and the same preferable groups as those of R in the formula (2) can be mentioned. m represents an integer of 3 to 6. When a gel of silica is treated with the compound (cyclic siloxane) represented by the formula (3), the following bonding will occur on the surface of the silica framework in the gel:

$$(\equiv Si\text{-}O\text{-})_2SiR^6R^7.$$

In this way, silanol groups may be silylated by the cyclic siloxanes of the above formula (3) as well, and thereby hydrophobization may be carried out.

Specific examples of the cyclic siloxanes represented by the above formula (3) include hexamethylcyclotrisiloxane, octamethylcyclotetrasiloxane, and decamethylcyclopentasiloxane.

The hydrophobic aerogels of silica silylate may be prepared by producing a sol of silica, turning the sol into a gel, aging the gel, washing the aged gel, replacing water in the washed gel with a solvent, treating the gel with a hydrophobicizing agent, and dying the hydrophobicized silica.

The hydrophobic aerogels of silica silylate may have a specific surface area determined by BET method of 200 $m^2/g$ or more, preferably 400 $m^2/g$ or more, and more preferably 500 $m^2/g$ or more, and may have a specific surface area determined by BET method of 1,200 $m^2/g$ or less, preferably 1,000 $m^2/g$ or less, and more preferably 800 $m^2/g$ or less.

The hydrophobic aerogels of silica silylate may have a pore volume determined by BJH method of 1 ml/g or more, preferably 2 ml/g or more, and more preferably 3 ml/g or more, and may have a pore volume determined by BJH method of 10 ml/g or less, preferably 8 ml/g or less, and more preferably 7 ml/g or less. The hydrophobic silica aerogel of silica silylate may have a peak pore radius determined by BJH method of 5 nm or more, preferably 10 nm or more, and more preferably 12 nm or more, and may have a peak pore radius determined by BJH method of 50 nm or less, preferably 40 nm or less, and more preferably 30 nm or less.

The "pore volume determined by BJH method" refers to a pore volume which derives from a pore having a pore radius of 1 nm to 100 nm obtained by analyzing, by the BJH method (Barrett, E. P.; Joyner, L. G.; Halenda, P. P., J. Am. Chem. Soc. 73, 373 (1951)), the adsorption isotherm of the nitrogen adsorption side obtained in the same manner as explained in the above "specific surface area determined by BET method". The "peak pore radius determined by BJT method" refers to a value of a pore radius which gives a peak in a pore distribution curve (volume distribution curve) which is plotted taking on the vertical axis differentiation of the cumulative pore volume by the logarithm of the pore radius obtained

by analyzing, by the BJH method, the adsorption isotherm of the nitrogen adsorption side obtained in the same manner as above, and taking the pore radius on the horizontal axis.

The hydrophobic aerogels of silica silylate may have an average particle size of 0.5 μm or more, preferably 1 μm or more, and more preferably 2 μm or more, and may have an average particle size by image analysis method of 30 μm or less, preferably 20 μm or less, and more preferably 15 μm or less.

The "average particle size" here can be measured by image analysis method. Specifically, the value of "average particle size" is an arithmetic mean of equivalent circle diameters which can be obtained by image analysis of a scanning electron microscope (SEM) image of, for example, no less than 2,000 aerogel particles observed at a magnification of 1,000 by secondary electron detection using a scanning electron microscope (SEM). The "equivalent circle diameter" of each aerogel particle is the diameter of a circle having an area equal to the area (projected area) of the aerogel particle in the image.

Preferably, the hydrophobic aerogels of silica silylate may have an oil-absorbing capacity, which can be measured at the wet point, as explained above, of 2 ml/g or more, preferably 3 ml/g or more, more preferably 4 ml/g or more, and most preferably from 5 ml/g or more, and may have an oil-absorbing capacity, measured at the wet point, of 12 ml/g or less, preferably 10 ml/g or less, more preferably 8 ml/g or less, and most preferably 7 ml/g or less.

The (d) filler comprises (d-1) at least one hydrophobic inorganic filler. The term "hydrophobic" means that the inorganic filler can be individually dispersed in an oil in such a manner that aggregates are not formed.

It may be preferable that the (d-1) hydrophobic inorganic filler be selected from hydrophobically-modified metal oxides, and more preferably selected from hydrophobic silicas. The term "hydrophobically-modified" means that, at least, the surface of metal oxides has been treated to be hydrophobic. In particular, silica silylate is most preferable.

The amount of the (d-1) hydrophobic inorganic filler(s) in the composition according to the present invention may be 0.1% by weight or more, preferably 0.2% by weight or more, and more preferably 0.3% by weight or more, relative to the total weight of the composition.

The amount of the (d-1) hydrophobic inorganic filler(s) in the composition according to the present invention is less than 2% by weight and may be 1.5% by weight or less, and preferably 1% by weight or less, relative to the total weight of the composition.

The amount of the (d-1) hydrophobic inorganic filler(s) in the composition according to the present invention may be from 0.1% to less than 2% by weight, preferably from 0.2% to 1.5% by weight, and more preferably from 0.3% to 1% by weight, relative to the total weight of the composition.

The (d) filler may comprises (d-2) at least one hydrophobic organic filler. The term "hydrophobic" means that the organic filler can be individually dispersed in an oil in such a manner that aggregates are not formed.

It may be preferable that the (d-2) hydrophobic organic filler be selected from hydrophobically-modified starches, and more preferably esterified starches. The term "hydrophobically-modified" means that, at least, the surface of starches has been treated to be hydrophobic.

The esterified starch preferably used in the present invention may be selected from the group consisting of phosphorylated starch, starch acetate, oxidized starch acetate, starch laurate, sodium starch phosphate, alkyl or alkenyl succinated starch, such as calcium starch octenylsuccinate, sodium starch octenylsuccinate, aluminum starch octenylsuccinate which is the starch esterified with octenylsuccinic anhydride and salts thereof, for example, sold under the name "Dry Flo Plus" from AKZO NOVEL, the potato starch esterified with a carboxymethyl group, sold under the name "Supramyl P 60" by Amylum, the corn starch esterified with a hydroxypropyl group, sold under the name "Merigel EF6" by Amylum, the corn starch esterified with dodecenylsuccinic anhydride (INCI name: CORN STARCH MODIFIED), and the potato starch esterified with halogenated methyl amino dipropionate acid (INCI name: POTATO STARCH MODIFIED).

The amount of the (d-2) hydrophobic organic filler(s) in the composition according to the present invention may be 1% by weight or more, preferably 5% by weight or more, and more preferably 10% by weight or more, relative to the total weight of the composition.

The amount of the (d-2) hydrophobic organic filler(s) in the composition according to the present invention may be 20% by weight or less, preferably 19% by weight or less, and more preferably 18% by weight or less, relative to the total weight of the composition.

The amount of the (d-2) hydrophobic organic filler(s) in the composition according to the present invention may be from 1% to 20% by weight, preferably from 5% to 19% by weight, and more preferably from 10% to 18% by weight, relative to the total weight of the composition.

The (d) filler may comprise (d-3) at least one hydrophilic filler, preferably at least one hydrophilic inorganic filler. The term "hydrophilic" means that the filler can be individually dispersed in water in such a manner that aggregates are not formed.

It may be preferable that the (d-3) hydrophilic filler be selected from metal oxides, and more preferably selected from silica, titanium oxide, zinc oxide and a mixture thereof. In particular, silica is most preferable.

The amount of the (d-3) hydrophilic filler(s) in the composition according to the present invention may be 1% by weight or more, preferably 5% by weight or more, and more preferably 10% by weight or more, relative to the total weight of

the composition.

The amount of the (d-3) hydrophilic filler(s) in the composition according to the present invention may be 20% by weight or less, preferably 19% by weight or less, and more preferably 18% by weight or less, relative to the total weight of the composition.

The amount of the (d-3) hydrophilic filler(s) in the composition according to the present invention may be from 1% to 20% by weight, preferably from 5% to 19% by weight, and more preferably from 10% to 18% by weight, relative to the total weight of the composition.

The amount of the (d) filler(s) in the composition according to the present invention may be 10% by weight or more, preferably 12% by weight or more, and more preferably 14% by weight or more, relative to the total weight of the composition.

The amount of the (d) filler(s) in the composition according to the present invention is less than 21% by weight and may be 20% by weight or less, preferably 19% by weight or less, and more preferably 18% by weight or less, relative to the total weight of the composition.

The amount of the (d) filler(s) in the composition according to the present invention may be from 10% to 20% by weight, preferably from 12% to 19% by weight, and more preferably from 14% to 18% by weight, relative to the total weight of the composition.

(Lipophilic Gelling Agent)

The composition according to the present invention comprises (e) at least one lipophilic gelling agent selected from organomodified clays. If two or more (e) lipophilic gelling agents are used, they may be the same or different.

The term "lipophilic gelling agent" means an agent, in a particulate form or not, which is capable of gelling the oil(s) in the composition according to the present invention.

The term "particulate" lipophilic gelling agent means a lipophilic gelling agent in the form of particles or of crystals (particulate or crystalline).

The composition according to the present invention comprises at least one lipophilic gelling agent which is preferably particulate.

According to the present invention, the (e) lipophilic gelling agent is selected from organomodified clays.

The clay denotes a material based on hydrated silicates and/or aluminosilicates, of lamellar structure.

The clays can be natural or synthetic and they are rendered lipophilic by treatment with an alkylammonium salt, such as a C10 to C22 ammonium chloride, in particular stearalkonium chloride or distearyldimethylammonium chloride.

They may be chosen from bentonites, in particular bentonites, hectorites and montmorillonites, beidellites, saponites, nontronites, sepiolites, biotites, attapulgites, vermiculites and zeolites.

Organomodified clays may be clays treated with compounds chosen especially from quaternary amines and tertiary amines. Organomodified clays that may be mentioned include organomodified bentonites, such as the product sold under the name Bentone 34 by the company Rheox, and organomodified hectorites such as the products sold under the names Bentone 27 and Bentone 38 by the company Rheox. Mention may be made especially of modified clays such as modified magnesium silicate (Bentone gel VS38 from Rheox), modified hectorites such as hectorite modified with a C10 to C22 fatty acid ammonium chloride, for instance hectorite modified with distearyldimethylammonium chloride, for instance the product sold under the name Bentone 38VCG by the company Elementis or the product sold under the name Bentone 38 CE by the company Rheox, or the product sold under the name Bentone Gel V5 5V by the company Elementis.

It may be preferable that the (e) lipophilic gelling agent be selected from organomodified bentonites, organomodified hectorites, and mixtures thereof.

It may be more preferable that the (e) lipophilic gelling agent be disteardimonium hectorite.

The amount of the (e) lipophilic gelling agent(s) in the composition according to the present invention is more than 2.5% by weight, and may be 3% by weight or more, preferably 4% by weight or more, and more preferably 5% by weight or more, relative to the total weight of the composition.

The amount of the (e) lipophilic gelling agent(s) in the composition according to the present invention is less than 10% by weight and may be 9.5% by weight or less, preferably 9% by weight or less, and more preferably 8.5% by weight or less, relative to the total weight of the composition.

The amount of the (e) lipophilic gelling agent(s) in the composition according to the present invention is more than 2.5% by weight and less than 10% by weight, may be from 3% to 9.5% by weight preferably from 4% to 9% by weight, and more preferably from 5% to 8.5% by weight, relative to the total weight of the composition.

(Total Amount Requirement)

The total amount of the (d) filler and the (e) lipophilic gelling agent in the composition according to the present invention is more than 18.5% by weight, and may be 19% by weight or more, preferably 20% by weight or more, and more

preferably 21% by weight or more, relative to the total weight of the composition.

The total amount of the (d) filler and the (e) lipophilic gelling agent in the composition according to the present invention may be 35% by weight or less, preferably 30% by weight or less, and more preferably 25% by weight or less, relative to the total weight of the composition.

The total amount of the (d) filler and the (e) lipophilic gelling agent in the composition according to the present invention may be from 19% to 35% by weight, preferably from 20% to 30% by weight, and more preferably from 21% to 25% by weight relative to the total weight of the composition.

(Optional Ingredients)

The composition according to the present invention may further comprise at least one optional ingredient usually used in the field of cosmetics, chosen, for example, film-forming polymers, solvents, dyes, pigments, UV filters, antioxidants, preserving agents, pH adjusting agents, cosmetic active agents, such as vitamins, moisturizers, emollients or collagen-protecting agents, and mixtures thereof.

The composition according to the present invention may include one or several cosmetically acceptable organic solvents, which may be alcohols: in particular monovalent alcohols such as ethyl alcohol, isopropyl alcohol, benzyl alcohol, and phenylethyl alcohol; diols such as ethylene glycol, caprylyl glycol, propylene glycol, and butylene glycol; other polyols such as glycerol, sugar, and sugar alcohols; and ethers such as ethylene glycol monomethyl, monoethyl, and monobutyl ethers, propylene glycol monomethyl, monoethyl, and monobutyl ether, and butylene glycol monomethyl, monoethyl, and monobutyl ethers.

The organic solvent(s) may then be present in a concentration of from 0.01% to 30% by weight, preferably from 0.1% to 20% by weight, and more preferably from 1% to 15% by weight, relative to the total weight of the composition.

It is a matter of routine operations for a person skilled in the art to adjust the nature and amount of the above optional additives which may be present in the composition in accordance with the present invention such that the desired cosmetic properties are not thereby affected.

(Cosmetic Product)

The composition according to the present invention may be a cosmetic composition, preferably a cosmetic composition for keratin substances, more preferably a cosmetic composition for keratin fibers, and even more preferably a cosmetic composition for eyebrows.

The composition according to the present invention may be a makeup cosmetic composition (in particular, an eye-makeup cosmetic composition), and more preferably an eyebrow makeup composition.

The composition according to the present invention can be used for cosmetic treatments, preferably makeup, of keratin substances, preferably keratin fibers, and more preferably the eyebrows.

The composition according to the present invention be in the form of an anhydrous liquid.

The term "anhydrous" here means that the composition according to the present invention may contain only a very small amount of water, and preferably no water. Thus, the amount of water in the composition according to the present invention may be 3% by weight or less, preferably 1% by weight or less, and more preferably 0.1% by weight or less, relative to the total weight of the composition. It is particularly preferable that the composition according to the present invention contain no water as a distinct ingredient to be added intentionally. On the other hand, a small or trace amount of water may be present in the ingredient itself to be included in the composition according to the present invention.

The term "liquid" here means that the composition is fluidable at room temperature (25°C) under atmospheric pressure (760 mmHg), and has a measurable viscosity. Thus, a composition in the form of a powder does not correspond to the composition according to the present invention.

[Preparation]

The composition according to the present invention can be prepared by mixing the above-described essential and optional ingredients.

For example, the composition according to the present invention can be prepared by a process comprising the step of

mixing the ingredients (a) to (e), as well as optional other ingredients of the composition, with or without heating, preferably at a temperature of 70°C or more. It is preferable to further mix with any of the above-described optional ingredients.

[Process and Use]

The composition according to the present invention can be used for coating keratin substances, preferably

keratin fibers, and more preferably the eyebrows.

For example, the composition according to the present invention can be used by a process for coating keratin fibers, comprising the step of:

applying onto the keratin fibers the composition according to the present invention to form at least one coating on the keratin fibers, and
drying the coating.

Thus, the present invention also relates to a cosmetic process for keratin substances, preferably keratin fibers, and more preferably the eyebrows, comprising the steps of: applying the composition according to the present invention onto the keratin substances.

The cosmetic process according to the present invention can be used for, for example, making up keratin substances, preferably keratin fibers, and more preferably the eyebrows.

The present invention may also relate to a use of

(d) at least one filler comprising (d-1) at least one hydrophobic inorganic filler, and
(e) at least one lipophilic gelling agent selected from organomodified clays,
in an anhydrous liquid composition for keratin substances, preferably keratin fibers, and more preferably the eyebrows, comprising
(a) at least one silicone resin;
(b) at least one silicone gum; and
(c) at least one volatile hydrocarbon oil,
wherein
the amount of the (d) filler and the (e) lipophilic gelling agent in the composition is more than 18.5% by weight, relative to the total weight of the composition,
the amount of the (e) lipophilic gelling agent in the composition is more than 2.5% by weight and less than 10% by weight, relative to the total weight of the composition,
the amount of the (d) filler in the composition is less than 21% by weight, relative to the total weight of the composition, and
the amount of the (d-1) hydrophobic inorganic filler in the composition is less than 2% by weight relative to the total weight of the composition,
in order to make the composition stable and to provide excellent cosmetic effects such as a matte finish, good wear resistance, and a comfortable texture such as reduced stickiness of the makeup and smooth spreading during application.

EXAMPLES

The present invention will be described in a more detailed manner by way of examples. However, these examples should not be construed as limiting the scope of the present invention. The examples below are presented as non-limiting illustrations in the field of the present invention.

**Example 1 and Comparative Examples 1-19**

The cosmetic compositions for making up the eyebrows according to Example 1 and Comparative Examples 1-19 shown in Tables 1 and 2 were prepared by mixing the ingredients shown in Tables 1 and 2. The numerical values for the amounts of the ingredients shown in Tables 1 and 2 are all based on "% by weight" of raw materials.

The symbols "a", "b", "c", "d", "d-1" and "e" in Tables 1 and 2 correspond to those in the claims.

Table 1

| | Ingredients | Ex. 1 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 |
|---|---|---|---|---|---|---|---|---|---|---|---|---|
| c | Isododecane | qsp100 | qsp100 | qsp100 | qsp100 | qsp100 | qsp100 | qsp100 | qsp100 | qsp100 | qsp100 | qsp100 |
| b | Dimethicone (1,000,000 $mm^2/s$) | 10 | 10 | 10 | 10 | 10 | 10 | 10 | 10 | 10 | 10 | 10 |
| alc | Trimethylsiloxysilicate (75wt%) and Isododecane (25wt%) | 23.3 | 23.3 | 23.3 | 23.3 | 23.3 | 23.3 | 23.3 | 23.3 | 23.3 | 23.3 | 23.3 |
| | Pentylene Glycol | 1 | 1 | 1 | 1 | 1 | 1 | 1 | 1 | 1 | 1 | 1 |
| | Caprylyl Glycol | 0.5 | 0.5 | 0.5 | 0.5 | 0.5 | 0.5 | 0.5 | 0.5 | 0.5 | 0.5 | 0.5 |
| | Propylene Carbonate | 2.475 | 2.475 | 1.65 | 1.65 | 2.475 | 2.475 | 1.65 | 3.3 | 3.3 | 3.3 | 0.825 |
| c | C8-9 Isoparaffin | 5 | 5 | 5 | 5 | 5 | 5 | 5 | 5 | 5 | 5 | 5 |
| | Titanium Dioxide (and) Alumina (and) Isopropyl Titanium Triisostearate | 2.04 | 2.04 | 2.04 | 2.04 | 2.04 | 2.04 | 2.04 | 2.04 | 2.04 | 2.04 | 2.04 |
| | Iron Oxides (and) Isopropyl Titanium Triisostearate | 3.46 | 3.46 | 3.46 | 3.46 | 3.46 | 3.46 | 3.46 | 3.46 | 3.46 | 3.46 | 3.46 |
| | Dimethicone (50 cs) | 7.5 | 7.5 | 7.5 | 2.5 | - | 10 | 5 | 10 | 5 | 5 | 7.5 |
| | Dextrin Palmitate | 0.5 | 1 | 2 | 1.5 | 1.5 | 2 | - | - | 0.5 | 1 | 0.5 |
| e | Disteardimonium Hectorite | 7.5 | 7.5 | 5 | 5 | 7.5 | 7.5 | 5 | 10 | 10 | 10 | 2.5 |
| d | Silica | 15 | - | - | - | 10 | 5 | 10 | 20 | 15 | 10 | 20 |
| d (d-1) | Silica Silvlate | 0.5 | 0.5 | 0.5 | - | 1 | 1.5 | 1 | - | 1 | 1 | 0.5 |
| | 18.5 < (d + e) | Yes | No | No | No | No | No | No | Yes | Yes | Yes | Yes |
| | 2.5 < e < 10 | Yes | Yes | Yes | Yes | Yes | Yes | Yes | No | No | No | No |
| | d < 21 | Yes | Yes | Yes | Yes | Yes | Yes | Yes | Yes | Yes | Yes | Yes |
| | d-1 < 2 | Yes | Yes | Yes | Yes | Yes | Yes | Yes | Yes | Yes | Yes | Yes |
| | Smoothness | Fair | Good | Good | Good | Fair | Good | Good | Poor | Poor | Poor | Fair |
| | Matte Effects After Drying | Very Good | Poor | Poor | Poor | Very Good | Good | Very Good | Very Good | Very Good | Very Good | Very Good |
| | Gloss Value After Drying | 0.8 | 6.2 | 6.3 | 12.1 | 0.9 | 1.2 | 0.7 | 0.8 | 0.8 | 0.8 | 0.7 |

(continued)

| Ingredients | Ex. 1 | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 | Comp. Ex. 9 | Comp. Ex. 10 |
|---|---|---|---|---|---|---|---|---|---|---|---|
| Stickiness After Drying | Not Sticky | Sticky | Sticky | Sticky | Not Sticky | Very Slightly Sticky | Not Sticky | Not Sticky | Not Sticky | Not Sticky | Not Sticky |
| Stability at 25 °C | Good | Fair | Poor | Poor | Poor | Poor | Fair | Very Good | Very Good | Good | Good |
| Stability at 45 °C | Good | Poor | Poor | Poor | Good | Goof | Poor | Very Good | Very Good | Very Good | Poor |
| Sebum Resistance | Good | Very Good | Good | Good | Very Good | Good | Good | Good | Good | Good | Good |
| Water Resistance | Very Good | Good | Good | Very Good | Very Good | Good | Good | Good | Very Good | Very Good | Very Good |

Table 2

| | Ingredients | Comp. Ex. 11 | Comp. Ex. 12 | Comp. Ex. 13 | Comp. Ex. 14 | Comp. Ex. 15 | Comp. Ex. 16 | Comp. Ex. 17 | Comp. Ex. 18 | Comp. Ex. 19 |
|---|---|---|---|---|---|---|---|---|---|---|
| c | Isododecane | qsp100 | qsp100 | qsp100 | qsp100 | qsp100 | qsp100 | qsp100 | qsp100 | qsp100 |
| b | Dimethicone (1,000,000 $mm^2/s$) | 10 | 10 | 10 | 10 | 10 | 10 | 10 | 10 | 10 |
| alc | Trimethylsiloxysilicate (75wt%) and Isododecane (25wt%) | 23.3 | 23.3 | 23.3 | 23.3 | 23.3 | 23.3 | 23.3 | 23.3 | 23.3 |
| | Pentylene Glycol | 1 | 1 | 1 | 1 | 1 | 1 | 1 | 1 | 1 |
| | Caprylyl Glycol | 0.5 | 0.5 | 0.5 | 0.5 | 0.5 | 0.5 | 0.5 | 0.5 | 0.5 |
| | Propylene Carbonate | 0.825 | - | - | 3.3 | 3.3 | - | 1.65 | 2.475 | 2.475 |
| c | C8-9 Isoparaffin | 5 | 5 | 5 | 5 | 5 | 5 | 5 | 5 | 5 |
| | Titanium Dioxide (and) Alumina (and) Isopropyl Titanium Triisostearate | 2.04 | 2.04 | 2.04 | 2.04 | 2.04 | 2.04 | 2.04 | 2.04 | 2.04 |
| | Iron Oxides (and) Isopropyl Titanium Triisostearate | 3.46 | 3.46 | 3.46 | 3.46 | 3.46 | 3.46 | 3.46 | 3.46 | 3.46 |
| | Dimethicone (50 cs) | - | 7.5 | 2.5 | 2.5 | 5 | 5 | 7.5 | 5 | 7.5 |
| | Dextrin Palmitate | 2 | 2 | 1.5 | 0.5 | 1 | 0.5 | 2 | 1 | 0.5 |
| e | Disteardimonium Hectorite | 2.5 | - | - | 10 | 10 | - | 5 | 7.5 | 7.5 |
| d | Silica | 5 | - | 5 | - | - | 10 | 20 | 15 | 10 |
| d (d-1) | Silica Silylate | 0.5 | 0.5 | 0.5 | 1 | - | 1.5 | 1 | 2 | 2 |
| | 18.5 < (d + e) | No | No | No | No | No | No | Yes | Yes | Yes |
| | 2.5 < e < 10 | No | No | No | No | No | No | Yes | Yes | Yes |
| | d < 21 | Yes | Yes | Yes | Yes | Yes | Yes | No | Yes | Yes |
| | d-1 < 2 | Yes | Yes | Yes | Yes | Yes | Yes | Yes | No | No |
| | Smoothness | Good | Good | Good | Good | Good | Good | Poor | Poor | Poor |
| | Matte Effects After Drying | Very Good | Poor | Good | Good | Poor | Very Good | Very Good | Very Good | Very Good |
| | Gloss After Drying | 0.9 | 24.3 | 5.1 | 1.6 | 15.3 | 0.7 | 0.8 | 0.8 | 0.8 |

(continued)

| | Ingredients | Comp. Ex. 11 | Comp. Ex. 12 | Comp. Ex. 13 | Comp. Ex. 14 | Comp. Ex. 15 | Comp. Ex. 16 | Comp. Ex. 17 | Comp. Ex. 18 | Comp. Ex. 19 |
|---|---|---|---|---|---|---|---|---|---|---|
| Stickiness After Drying | | Not Sticky | Sticky | Sticky | Not Sticky | Sticky | Not Sticky | Not Sticky | Not Sticky | Not Sticky |
| Stability at 25 °C | | Fair | Poor | Poor | Good | Good | Poor | Very Good | Very Good | Very Good |
| Stability at 45 °C | | Poor | Poor | Poor | Poor | Fair | Poor | Good | Very Good | Very Good |
| Sebum Resistance | | Very Good | Very Good | Very Good | Good | Good | Very Good | Fair | Good | Good |
| Water Resistance | | Very Good | Poor | Very Good | Very Good | Very Good | Very Good | Very Good | Very Good | Very Good |

[Evaluations]

(Smoothness)

The cosmetic composition of each of Example 1 and Comparative Examples 1-19 was filled into a dip-in applicator package and evaluated in terms of smoothness during application on arm in accordance with the following criteria.

Good: Light to moderate smooth gliding was felt
Fair: Slightly less smooth gliding was felt, but acceptable
Poor: Unacceptable druggy texture was felt

The results are shown in Tables 1 and 2.

(Matte Effects After Drying)

A film with a thickness of 100 μm was prepared on contrast cards (white and black) by applying the cosmetic composition of each of Example 1 and Comparative Examples 1-19. The film was dried at room temperature for 30 minutes. Then, the matte effects on the film were evaluated by panelists in accordance with the following criteria.

Very Good: Totally matte without gloss
Good: Very slightly glossy
Poor: Glossy

The results are shown in Tables 1 and 2.

(Gloss After Drying)

A film with a thickness of 100 μm was prepared on contrast cards (white and black) by applying the cosmetic composition of each of Example 1 and Comparative Examples 1-19. The film was dried at room temperature for 30 minutes. Then, the gloss (reflected light on the cards) of the film was measured by a polarimetric camera. The average value of gloss on white and back backgrounds was used as a gloss value.
The results are shown in Tables 1 and 2.
Smaller gloss value means better matte effect. It is preferable that a gloss value be less than 6.0, and more preferably below 1.0.

(Stickiness After Drying)

A film with a thickness of 100 μm was prepared on contrast cards (white and black) by applying the cosmetic composition of each of Example 1 and Comparative Examples 1-19. The film was dried at room temperature for 30 minutes.
The stickiness of the dried film was evaluated by panelists in accordance with the following criteria:

Not Sticky: Completely non adhesive sensation is felt when touching surface of film by finger
Very Slightly Sticky (acceptable): Very slightly adhesive, but non-significant sensation is felt when touching film by finger
Slightly: Clear adhesive sensation is felt when touching the film by finger

The results are shown in Tables 1 and 2.

(Stability at 25 °C or 45 °C)

The cosmetic composition of each of Example 1 and Comparative Examples 1-19 was filled into a 50 ml glass jar and maintained at 25 °C or 45 °C for 8 weeks.
Then, the presence or absence of separated oil on the top surface of each composition was checked. If separated oil was present, the phase separation rate was calculated by the following equation:

$$\text{Separation Rate (\%)} = A / B \times 100$$

A: the height of separated oil on top (mm),
B: the height of total bulk composition including the separated oil (mm)

The stability of each cosmetic composition was evaluated in accordance with the following criteria:

Very Good: No separated oil or very slightly oil separation (unmeasurable)
Good: Slight oil separation with less than 5% separation rate
Fair: Oil separation with 5% or more and less than 6% separation rate
Poor: Large oil separation with 6% or more separation rate
Very Poor: Complete separation

The results are shown in Tables 1 and 2.

(Sebum Resistance)

The cosmetic composition of each of Example 1 and Comparative Examples 1-19 was filled into a dip-in applicator package with a flocked applicator, was applied onto a white Bio Skin sheet by Beaulax, and was dried at room temperature for 30 minutes to form a dry film. Then, an artificial sebum* was dropped on the film and rubbed with 30 stokes by a finger at room temperature (1 stroke/sec). The level of smudge was evaluated in accordance with the following criteria:

Very Good: Almost no smudge
Good: Very slightly smudged
Fair: Slightly smudged (acceptable)
Poor: Smudged heavily and partially or completely faded
* Composition of Artificial Sebum:
28.7 wt% triisostearin, 28 wt% oleic acid, 22.9 wt% oleyl erucate, 13.7 wt% hydrogenated polyisobutene, and 6.7 wt% of octyldodecanol

The results are shown in Tables 1 and 2.

(Water resistance)

The cosmetic composition of each of Example 1 and Comparative Examples 1-19 was filled into a dip-in applicator package with a flocked applicator, was applied onto a white Bio Skin sheet by Beaulax, and was dried at room temperature for 30 minutes to form a dry film. Then, water was dropped on the film and rubbed with 30 stokes by a finger at room temperature (1 stroke/sec). The level of smudge was evaluated in accordance with the following criteria:

Very Good: Almost no smudge
Good: Very slightly smudged
Poor: Creased or faded

The results are shown in Tables 1 and 2.

(Summary)

As shown in Tables 1 and 2, the cosmetic composition according to Example 1 which corresponds to the present invention was stable and was able to provide matte and sebum/water resistant cosmetic effects as well as a comfortable texture such as smoothness when applying and non-stickiness after drying.

On the other hand, the cosmetic compositions according to Comparative Examples 1-19 which do not satisfy any of the amount requirements of

the amount of the (d) filler and the (e) lipophilic gelling agent is more than 18.5% by weight,
the amount of the (e) lipophilic gelling agent is more than 2.5% by weight and less than 10% by weight,
the amount of the (d) filler is less than 21% by weight, and
the amount of the (d-1) hydrophobic inorganic filler is less than 2% by weight

was not stable, or could not provide matte or sebum/water resistant cosmetic effects or excellent feeling to use such as smoothness when applying and non-stickiness after drying.

**Examples 2 and 3, and Comparative Example 20**

The cosmetic compositions for making up eyebrows according to Examples 2 and 3, and Comparative Example 20 shown in Table 3 were prepared by mixing the ingredients shown in Table 3. The numerical values for the amounts of the ingredients shown in Table 3 are all based on "% by weight" of raw materials.

The symbols "a", "b", "c", "d", "d-1" and "e" in Table 3 correspond to those in the claims.

Table 3

| | Ingredients | Ex. 2 | Ex. 3 | Comp. Ex. 20 |
|---|---|---|---|---|
| c | Isododecane | qsp100 | qsp100 | qsp100 |
| b | Dimethicone (1,000,000 $mm^2/s$) | 9.9 | - | - |
| b/c | Dimethicone (1,000,000 $mm^2/s$) (75wt%) and Isododecane (25wt%) | - | 39.6 | - |
| | Dimethicone (10 cs) | - | - | 9.9 |
| a | Trimethylsiloxysilicate | 13.5 | 13.5 | 13.5 |
| | Pentylene Glycol | 1 | 1 | 1 |
| | Caprylyl Glycol | 0.5 | 0.5 | 0.5 |
| | Propylene Carbonate | 2.64 | 2.64 | 2.64 |
| c | C8-9 Isoparaffin | 1 | 1 | 1 |
| | Titanium Dioxide (and) Alumina (and) Isopropyl Titanium Triisostearate | 2.04 | 2.04 | 2.04 |
| | Iron Oxides (and) Isopropyl Titanium Triisostearate | 3.46 | 3.46 | 3.46 |
| e | Disteardimonium Hectorite | 8 | 8 | 8 |
| d | Aluminum Starch Octenylsuccinate | 15.5 | 15.5 | 15.5 |
| d (d-1) | Silica Silylate | 0.5 | 0.5 | 0.5 |
| | 18.5 < (d + e) | Yes | Yes | Yes |
| | 2.5 < e < 10 | Yes | Yes | Yes |
| | d < 21 | Yes | Yes | Yes |
| | d-1 < 2 | Yes | Yes | Yes |
| | Smoothness | Good | Good | Good |
| | Gloss After Drying | 0.7 | 0.7 | 0.9 |
| | Stickiness After Drying | Not Sticky | Not Sticky | Slightly Sticky |
| | Stability at 25 °C | Good | Good | NA |
| | Stability at 45 °C | Good | Good | NA |
| | Sebum Resistance | Good | Good | Poor |
| | Water Resistance | Very Good | Very Good | Poor |
| NA: Not Available | | | | |

[Evaluations]

"Smoothness", "Gloss After Drying", "Stickiness After Drying", "Stability at 25 °C", "Stability at 45 °C", "Sebum Resistance" and "Water Resistance" were evaluated in accordance with the protocols as used for the evaluations of Example 1 and Comparative Examples 1-19.

The results are shown in Table 3.

As shown in Table 3, the cosmetic compositions according to Examples 2 and 3 which correspond to the present invention were stable, and was able to provide matte and sebum/water resistant cosmetic effects as well as a comfortable texture such as smoothness when applying and non-stickiness after drying.

The composition according to Example 2 and the composition according to Example 3 are similar. However, they are different in the point that the dimethicone with a dynamic viscosity of 1,000,000 $mm^2/s$ is diluted with isododecane for Example 3, while the dimethicone with a dynamic viscosity of 1,000,000 $mm^2/s$ is not diluted for Example 2. The production of the composition according to Example 3 was easier than that of the composition according to Example 2 because the handling of the dimethicone was improved in Example 3.

On the other hand, the cosmetic composition according to Comparative Example 20 which does not include any silicone gum could not provide sebum/water resistant cosmetic effects or a comfortable texture such as non-stickiness after drying.

## Claims

1. An anhydrous liquid composition for keratin substances, preferably for keratin fibers, and more preferably for eyebrows, comprising:

    (a) at least one silicone resin;
    (b) at least one silicone gum;
    (c) at least one volatile hydrocarbon oil;
    (d) at least one filler; and
    (e) at least one lipophilic gelling agent selected from organomodified clays,

    wherein

    the (d) filler comprises
    (d-1) at least one hydrophobic inorganic filler, preferably selected from hydrophobically-modified metal oxides, and more preferably selected from hydrophobic silicas,
    and
    wherein
    the total amount of the (d) filler and the (e) lipophilic gelling agent in the composition is more than 18.5% by weight, relative to the total weight of the composition,
    the amount of the (e) lipophilic gelling agent in the composition is more than 2.5% by weight and less than 10% by weight, relative to the total weight of the composition,
    the amount of the (d) filler in the composition is less than 21% by weight, relative to the total weight of the composition, and
    the amount of the (d-1) hydrophobic inorganic filler in the composition is less than 2% by weight relative to the total weight of the composition.

2. The composition according to Claim 1, wherein the (a) silicone resin is selected from MQ resins.

3. The composition according to Claim 1 or 2, wherein the (a) silicone resin is present in an amount of from 1% to 30% by weight, preferably 5% to 25% by weight, and more preferably 10% to 20% by weight, relative to the total weight of the composition.

4. The composition according to any one of Claims 1 to 3, wherein the (b) silicone gum is selected from polydimethylsiloxane gums.

5. The composition according to any one of Claims 1 to 4, wherein the (b) silicone gum has a dynamic viscosity at 25 °C of 400,000 $mm^2/s$ or more, preferably 600,000 $mm^2/s$ or more, and more preferably 800,000 $mm^2/s$ or more.

6. The composition according to any one of Claims 1 to 5, wherein the (b) silicone gum is present in an amount of from 1% to 25% by weight, preferably 3% to 20% by weight, and more preferably 5% to 15% by weight, relative to the total weight of the composition.

7. The composition according to any one of Claims 1 to 6, wherein the (c) volatile hydrocarbon oil is selected from

isododecane, C8-9 isoparaffin and a mixture thereof.

**8.** The composition according to any one of Claims 1 to 7, wherein the (c) volatile hydrocarbon oil is present in an amount of from 10% to 60% by weight, preferably from 15% to 55% by weight, and more preferably 20% to 50% by weight, relative to the total weight of the composition.

**9.** The composition according to any one of Claims 1 to 8, wherein the (d) filler is present in an amount of from 10% to 20% by weight, preferably from 12% to 19% by weight and more preferably from 14% to 18% by weight, relative to the total weight of the composition.

**10.** The composition according to any one of Claims 1 to 9, wherein the (d) filler further comprises (d-2) at least one hydrophobic organic filler, preferably selected from hydrophobically-modified starches, and more preferably selected from esterified starches.

**11.** The composition according to any one of Claims 1 to 10, wherein the (d-1) hydrophobic inorganic filler is present in an amount of from 0.1% to less than 2% by weight, preferably from 0.2% to 1.5% by weight, and more preferably from 0.3% to 1% by weight, relative to the total weight of the composition.

**12.** The composition according to any one of Claims 1 to 11, wherein the (e) lipophilic gelling agent is selected from organomodified bentonites, organomodified hectorites, and mixtures thereof.

**13.** The composition according to any one of Claims 1 to 12, wherein the (e) lipophilic gelling agent is present in an amount of from 3% to 9.5% by weight, preferably from 4% to 9% by weight, and more preferably from 5% to 8.5% by weight relative to the total weight of the composition.

**14.** The composition according to any one of Claims 1 to 13, wherein the (d) filler and the (e) lipophilic gelling agent are present in a total amount of from 19% to 35% by weight, preferably from 20% to 30% by weight, and more preferably from 21% to 25% by weight relative to the total weight of the composition.

**15.** A cosmetic process for keratin substances, preferably keratin fibers, and more preferably the eyebrows, comprising the steps of:
applying the composition according to any one of Claims 1 to 14 onto the keratin substances.

## Patentansprüche

**1.** Wasserfreie flüssige Zusammensetzung für Keratinsubstanzen, vorzugsweise für Keratinfasern, und bevorzugter für Augenbrauen, umfassend:

(a) mindestens ein Silikonharz;
(b) mindestens ein Silikongummi;
(c) mindestens ein flüchtiges Kohlenwasserstofföl;
(d) mindestens einen Füllstoff; und
(e) mindestens ein lipophiles Geliermittel, das ausgewählt ist aus organomodifizierten Tonen, wobei

der Füllstoff (d) Folgendes umfasst

(d-1) mindestens einen hydrophoben anorganischen Füllstoff, der vorzugsweise ausgewählt ist aus hydrophob modifizierten Metalloxiden und bevorzugter aus hydrophoben Silikaten,
und
wobei
die Gesamtmenge des (d) Füllstoffs und des (e) lipophilen Geliermittels in der Zusammensetzung mehr als 18,5 Gewichts-% bezogen auf das Gesamtgewicht der Zusammensetzung ist,
die Menge des (e) lipophilen Geliermittels in der Zusammensetzung mehr als 2,5 Gewichts-% und weniger als 10 Gewichts-% bezogen auf das Gesamtgewicht der Zusammensetzung ist,
die Menge des (d) Füllstoffs in der Zusammensetzung weniger als 21 Gewichts-% bezogen auf das Gesamtgewicht der Zusammensetzung ist, und
die Menge des (d-1) hydrophoben anorganischen Füllstoffs in der Zusammensetzung weniger als 2 Gewichts-%

bezogen auf das Gesamtgewicht der Zusammensetzung ist.

2. Zusammensetzung nach Anspruch 1, wobei das (a) Silikonharz ausgewählt ist aus MQ-Harzen.

3. Zusammensetzung nach Anspruch 1 oder 2, wobei das (a) Silikonharz in einer Menge von 1 bis 30 Gewichts-%, vorzugsweise 5 bis 25 Gewichts-% und bevorzugter 10 bis 20 Gewichts-% bezogen auf das Gesamtgewicht der Zusammensetzung vorhanden ist.

4. Zusammensetzung nach einem der Ansprüche 1 bis 3, wobei der (b) Silikongummi ausgewählt ist aus Polydimethylsiloxan-Gummis.

5. Zusammensetzung nach einem der Ansprüche 1 bis 4, wobei der (b) Silikongummi eine dynamische Viskosität bei 25 °C von 400.000 $mm^2/s$ oder mehr, vorzugsweise 600.000 $mm^2/s$ oder mehr und bevorzugter 800.000 $mm^2/s$ oder mehr aufweist.

6. Zusammensetzung nach einem der Ansprüche 1 bis 5, wobei der (b) Silikongummi in einer Menge von 1 bis 25 Gewichts-%, vorzugsweise 3 bis 20 Gewichts-% und bevorzugter 5 bis 15 Gewichts-% bezogen auf das Gesamtgewicht der Zusammensetzung vorhanden ist.

7. Zusammensetzung nach einem der Ansprüche 1 bis 6, wobei das (c) flüchtige Kohlenwasserstofföl ausgewählt ist aus Isododecan, C8-9-Isoparaffin und einem Gemisch davon.

8. Zusammensetzung nach einem der Ansprüche 1 bis 7, wobei das (c) flüchtige Kohlenwasserstofföl in einer Menge von 10 bis 60 Gewichts-%, vorzugsweise von 15 bis 55 Gewichts-% und bevorzugter von 20 bis 50 Gewichts-% bezogen auf das Gesamtgewicht der Zusammensetzung vorhanden ist.

9. Zusammensetzung nach einem der Ansprüche 1 bis 8, wobei der (d) Füllstoff in einer Menge von 10 bis 20 Gewichts-%, vorzugsweise von 12 bis 19 Gewichts-% und bevorzugter von 14 bis 18 Gewichts-% bezogen auf das Gesamtgewicht der Zusammensetzung vorhanden ist.

10. Zusammensetzung nach einem der Ansprüche 1 bis 9, wobei der (d) Füllstoff ferner (d-2) mindestens einen hydrophoben organischen Füllstoff umfasst, der vorzugsweise ausgewählt ist aus hydrophob modifizierten Stärken und bevorzugter aus veresterten Stärken.

11. Zusammensetzung nach einem der Ansprüche 1 bis 10, wobei der (d-1) hydrophobe anorganische Füllstoff in einer Menge von 0,1 bis weniger als 2 Gewichts-%, vorzugsweise von 0,2 bis 1,5 Gewichts-% und bevorzugter von 0,3 bis 1 Gewichts-% bezogen auf das Gesamtgewicht der Zusammensetzung vorhanden ist.

12. Zusammensetzung nach einem der Ansprüche 1 bis 11, wobei das (e) lipophile Geliermittel ausgewählt ist aus organomodifizierten Bentoniten, organomodifizierten Hectoriten und Gemischen davon.

13. Zusammensetzung nach einem der Ansprüche 1 bis 12, wobei das (e) lipophile Geliermittel in einer Menge von 3 bis 9,5 Gewichts-%, vorzugsweise von 4 bis 9 Gewichts-% und bevorzugter von 5 bis 8,5 Gewichts-% bezogen auf das Gesamtgewicht der Zusammensetzung vorhanden ist.

14. Zusammensetzung nach einem der Ansprüche 1 bis 13, wobei der (d) Füllstoff und das (e) lipophile Geliermittel in einer Gesamtmenge von 19 bis 35 Gewichts-%, vorzugsweise von 20 bis 30 Gewichts-% und bevorzugter von 21 bis 25 Gewichts-% bezogen auf das Gesamtgewicht der Zusammensetzung vorhanden sind.

15. Kosmetisches Verfahren für Keratinsubstanzen, vorzugsweise Keratinfasern, und bevorzugter für die Augenbrauen, umfassend die folgenden Schritte:
Auftragen der Zusammensetzung nach einem der Ansprüche 1 bis 14 auf die Keratinsubstanzen.

## Revendications

1. Composition liquide anhydre pour les substances kératiniques, de préférence pour les fibres kératiniques, et plus préférentiellement pour les sourcils, comprenant :

(a) au moins une résine de silicone ;
(b) au moins une gomme de silicone ;
(c) au moins une huile hydrocarbonée volatile ;
(d) au moins une charge ; et
(e) au moins un agent gélifiant lipophile choisi parmi les argiles organomodifiées, dans laquelle

la charge (d) comprend

(d-1) au moins une charge inorganique hydrophobe, de préférence choisie parmi les oxydes métalliques modifiés de manière hydrophobe, et plus préférentiellement parmi les silices hydrophobes,
et
dans laquelle
la quantité totale de la charge (d) et de l'agent gélifiant lipophile (e) dans la composition est supérieure à 18,5 % en poids, par rapport au poids total de la composition,
la quantité de l'agent gélifiant lipophile (e) dans la composition est supérieure à 2,5 % en poids et inférieure à 10 % en poids, par rapport au poids total de la composition, la quantité de la charge (d) dans la composition est inférieure à 21 % en poids, par rapport au poids total de la composition, et
la quantité de la charge inorganique hydrophobe (d-1) dans la composition est inférieure à 2 % en poids par rapport au poids total de la composition.

**2.** Composition selon la revendication 1, dans laquelle la résine de silicone (a) est choisie parmi les résines MQ.

**3.** Composition selon la revendication 1 ou 2, dans laquelle la résine de silicone (a) est présente dans une quantité 1 % à 30 % en poids, de préférence de 5 % à 25 % en poids, et plus préférentiellement de 10 % à 20 % en poids, par rapport au poids total de la composition.

**4.** Composition selon l'une quelconque des revendications 1 à 3, dans laquelle la gomme de silicone (b) est choisie parmi les gommes de polydiméthylsiloxane.

**5.** Composition selon l'une quelconque des revendications 1 à 4, dans laquelle la gomme de silicone (b) a une viscosité dynamique à 25 °C de 400000 $mm^2/s$ ou plus, de préférence de 600000 $mm^2/s$ ou plus, et plus préférentiellement de 800000 $mm^2/s$ ou plus.

**6.** Composition selon l'une quelconque des revendications 1 à 5, dans laquelle la gomme de silicone (b) est présente dans une quantité de 1 % à 25 % en poids, de préférence de 3 % à 20 % en poids, et de plus préférentiellement de 5 % à 15 % en poids, par rapport au poids total de la composition.

**7.** Composition selon l'une quelconque des revendications 1 à 6, dans laquelle l'huile hydrocarbonée volatile (c) est choisie parmi l'isododécane, l'isoparaffine C8-9 et un mélange de ceux-ci.

**8.** Composition selon l'une quelconque des revendications 1 à 7, dans laquelle l'huile hydrocarbonée volatile (c) est présente dans une quantité de 10 % à 60 % en poids, de préférence de 15 % à 55 % en poids, et de plus préférentiellement de 20 % à 50 % en poids, par rapport au poids total de la composition.

**9.** Composition selon l'une quelconque des revendications 1 à 8, dans laquelle la charge (d) est présente dans une quantité de 10 % à 20 % en poids, de préférence de 12 % à 19 % en poids, et de plus préférentiellement de 14 % à 18 % en poids, par rapport au poids total de la composition.

**10.** Composition selon l'une quelconque des revendications 1 à 9, dans laquelle la charge (d) comprend en outre (d-2) au moins une charge organique hydrophobe, de préférence choisie parmi les amidons modifiés de manière hydrophobe, et plus préférentiellement choisie parmi les amidons estérifiés.

**11.** Composition selon l'une quelconque des revendications 1 à 10, dans laquelle la charge inorganique hydrophobe (d-1) est présente dans une quantité de 0,1 % à moins de 2 % en poids, de préférence de 0,2 % à 1,5 % en poids, et de plus préférentiellement de 0,3 % à 1 % en poids, par rapport au poids total de la composition.

**12.** Composition selon l'une quelconque des revendications 1 à 11, dans laquelle l'agent gélifiant lipophile (e) est choisi parmi les bentonites organomodifiées, les hectorites organomodifiées et les mélanges de celles-ci.

**13.** Composition selon l'une quelconque des revendications 1 à 12, dans laquelle l'agent gélifiant lipophile (e) est présent dans une quantité de 3 % à 9,5 % en poids, de préférence de 4 % à 9 % en poids, et de plus préférentiellement de 5 % à 8,5 % en poids, par rapport au poids total de la composition.

**14.** Composition selon l'une quelconque des revendications 1 à 13, dans laquelle la charge (d) et l'agent gélifiant lipophile (e) sont présents dans une quantité totale de 19 % à 35 % en poids, de préférence de 20 % à 30 % en poids, et de plus préférentiellement de 21 % à 25 % en poids, par rapport au poids total de la composition.

**15.** Procédé cosmétique pour les substances kératiniques, de préférence les fibres kératiniques, et plus préférentiellement les sourcils, comprenant les étapes suivantes :
application sur les substances kératiniques de la composition selon l'une quelconque des revendications 1 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6074654 A
- US 6139823 A
- US 6340466 B
- US 6406683 B
- US 6071503 A
- US 6019962 A
- US 6464964 B
- US 7160550 B
- FR 2939034
- US 2676182 A
- US 3627851 A
- US 3772247 A
- US 5248739 A
- US 5082706 A
- US 5319040 A
- US 5302685 A
- US 4935484 A
- US 5817302 A
- US 5110890 A
- WO 2005075542 A
- DE 102008012457
- US 7470725 B
- JP 2014088307 A
- JP 2014218433 A
- JP 2018177620 A

### Non-patent literature cited in the description

- Encyclopedia of Polymer Science and Engineering. John Wiley & Sons, 1989, vol. 15, 265-270
- **BRINKER C.J.** ; **SCHERER G.W.** Sol-Gel Science. Academic Press, 1990
- *Journal of the American Chemical Society*, February 1938, vol. 60, 309
- **VAN DE HULST, H.C.** Light Scattering by Small Particles. Wiley, 1957
- **BARRETT, E. P.** ; **JOYNER, L. G.** ; **HALENDA, P. P.** *J. Am. Chem. Soc.*, 1951, vol. 73, 373